# FASCICULE DE BREVET EUROPEEN

(11) **EP 0 598 635 B1**
(45) Date de publication et mention de la délivrance du brevet: **01.09.1999**
(21) Numéro de dépôt: 93402585.9
(22) Date de dépôt: 21.10.1993
(51) Int. Cl.: A61F 2/02, A61M 25/00, A61M 39/02

(54) **Dispositif amovible de filtration sanguine, à rigidité variable, implantable dans le corps d'un patient**
Abnehmbare und in den Körper eines Patienten implantierbare Blutfiltrationsvorrichtung mit veränderlicher Steifheit
Removable blood filtration device with variable stiffness implantable in the body of a patient

(30) Priorité: 19.11.1992 FR 9213909
(43) Date de publication de la demande: 25.05.1994
(73) Titulaire: B. BRAUN CELSA, 92100 Boulogne-Billancourt (FR)
(72) Inventeur: Cottenceau, Jean-Philippe, F-92160 Antony (FR); Chevillon, Gérard, F-92120 Montrouge (FR)
(74) Mandataire: Lerner, François

(56) Documents cités:
- EP-A- 0 229 729
- EP-A- 0 448 886
- WO-A-90/10466
- WO-A-92/07507
- DE-A- 3 203 410
- FR-A- 2 643 250
- US-A- 4 983 169
- US-A- 5 085 649
- US-A- 5 152 777

## Description

L'invention se rapporte au domaine des unités ou dispositifs de filtration sanguine.

Plus précisément l'invention concerne, dans ce domaine, les dispositifs implantables dans le corps d'un patient, comprenant un filtre sanguin pouvant être mis en place dans un vaisseau, au bout d'un cathéter ou d'un filament.

Ce type de dispositif est souvent dénommé "filtre temporaire".

Des exemples de réalisation et d'utilisation de tels dispositifs sont notamment décrits aux brevets FR-A-2 580 504 ou FR-A-2 643 250.

Toutefois, à la connaissance du demandeur, il n'a pas jusqu'à présent été tenu compte, notamment sur ce type de dispositif, d'un problème qui peut s'avérer important, à savoir la migration du filtre.

Or, si pour faire circuler un produit, ou du moins pour tenir le filtre, on utilise un cathéter et que celui-ci est réalisé en matériau souple favorable à son glissement le long de la voie d'accès au vaisseau, des effets de boucles peuvent apparaître sur sa longueur, ce qui risque de faire remonter progressivement l'unité vers le coeur. Si par contre le cathéter utilisé est plus rigide et qu'il ne doit en conséquence normalement plus avoir tendance à s'enrouler en boucles, il risque par contre de ne plus amortir, voire de favoriser, la poussée que tend à exercer sur lui le filtre, le cathéter ayant alors tendance à tirer sur les tissus.

C'est en particulier pour résoudre ces problèmes de migration, que l'invention propose un dispositif implantable comprenant :
- un cathéter présentant une paroi entourant un axe et une longueur dans la direction de cet axe, avec une extrémité proximale et une extrémité distale opposée,
- un filtre sanguin fixé à l'extrémité distale du cathéter, le dispositif se caractérisant en ce que le cathéter utilisé est raidi de manière variable sur sa longueur, pour être plus raide vers son extrémité distale que vers son extrémité proximale.

Pour par ailleurs autoriser de préférence la possible injection d'un liquide, ou d'un produit traitant, jusqu'à la zone d'implantation du filtre (voire une éventuelle ponction sanguine), cette rigidité variable pourra être obtenue par une rigidification longitudinalement évolutive de la paroi du cathéter, à l'extérieur de son conduit interne que l'on maintiendra ainsi libre pour le passage dudit liquide, dans un sens ou dans l'autre.

Ainsi, le cathéter va autoriser la mise en place, par voie souscutanée, d'un moyen implantable de distribution du produit traitant.

Pour en revenir brièvement à la rigidification variable du cathéter, on notera que celle-ci pourra être obtenue en particulier de deux manières : par rigidification interne de la paroi elle-même de ce cathéter, sans moyen annexe rapporté (par exemple en faisant varier l'épaisseur de cette paroi ou en utilisant des tronçons télescopiques), une autre solution étant d'utiliser des moyens de rigidification annexes pouvant notamment être noyés dans la paroi du cathéter.

A toutes fins utiles, on notera que l'extrémité "distale" du dispositif désigne l'extrémité à laquelle est fixé le filtre sanguin, l'extrémité "proximale" étant bien entendu l'extrémité opposée située le plus près de la surface de la peau, à proximité du débouché de la voie d'accès menant au vaisseau considéré.

D'autres caractéristiques et avantages de l'invention apparaîtront encore de la description qui va suivre faite en référence aux dessins annexés dans lesquels :
- la figure 1 est une vue schématique d'ensemble d'une unité de filtration sanguine amovible équipée d'une chambre implantable,
- la figure 2 est une vue en coupe médiane du détail repéré II sur la figure 1,
- la figure 3 montre une vue intérieure de la chambre implantable ainsi que la manière dont on peut l'utiliser,
- la figure 4 est une vue partielle en coupe longitudinale d'un cathéter à rigidité variable équipé d'un filtre,
- la figure 5 présente une variante de réalisation, - la figure 6 montre, selon la même vue que la figure 5, une autre variante de réalisation du cathéter avec son filtre,
- la figure 7 montre le moyen de rigidification utilisé dans la réalisation de la figure 6,
- les figures 8, 9 et 10 sont trois vues en coupe selon les lignes respectivement VIII-VIII, IX-IX et X-X de la figure 6,
- la figure 11 montre en coupe longitudinale une quatrième variante de réalisation du cathéter,
- la figure 12 est une vue agrandie du détail repéré XII sur la figure 11,
- les figures 13 et 14 montrent deux autres possibilités de rigidification variable du cathéter,
- et les figures 15, 16 et 17 montrent les principaux moyens utilisés pour la mise en place, ou le retrait, de l'unité de filtration de I'invention.

L'un des intérêts de l'utilisation, conformément à l'invention, d'un cathéter à rigidité variable, en liaison avec un filtre sanguin, étant donc de permettre l'emploi d'une chambre implantable sous la peau d'un patient devant par exemple subir une thrombolyse, on ne décrira ci-après l'invention que dans le cadre d'une telle application, même s'il doit être clair qu'éventuellement l'extrémité proximale du cathéter pourrait n'être pas reliée à une telle chambre.

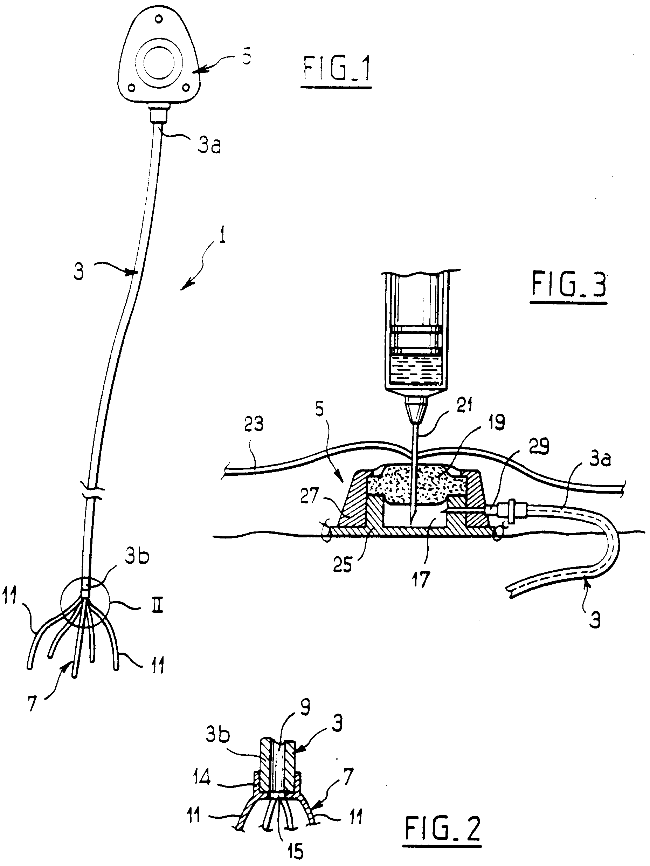

L'unité 1 de la figure 1 comprend un cathéter 3 sécable, à rigidité variable, relié du côté de son extrémité proximale 3a, à une chambre implantable 5, le cathéter étant en outre relié fixement du côté de son extrémité distale 3b, à un filtre sanguin 7.

La fixation du filtre 7 à l'extrémité distale 3b s'effectue de telle manière que le cathéter ne soit aucunement bouché (cette caractéristique ne doit toutefois pas être considérée comme indispensable).

Le filtre comporte, dans l'exemple illustré, six bras 11 autoexpansibles radialement, pour constituer une sorte d'ombrelle, de telle manière que dans leur état expansé (illustré par exemple sur la figure 1), les bras prennent une forme sensiblement conique pour pouvoir épouser par leur extrémité libre la paroi correspondante du vaisseau où le filtre doit être implanté, sans pour autant s'y accrocher définitivement.

A l'opposé de leur extrémité libre, les pattes du filtre sont réunies ensemble à l'endroit d'un sommet ou tête 14 traversé en son centre par un passage 15 d'une section comparable à celle du conduit 9.

Le filtre 7 étant normalement prévu pour être réalisé en métal, par exemple en acier au cobalt, il pourra être fixé au cathéter par sertissage et/ou collage de sa tête 14, de telle manière que l'orifice 15 vienne dans le prolongement du conduit 9 du cathéter.

Sur la figure 3, on a représenté un mode possible de réalisation de la chambre implantable 5.

Sur cette figure, on peut constater que la chambre est constituée à la manière d'une capsule étanche, renfermant une chambre intérieure 17 fermée en partie supérieure par une paroi auto-obturante 19 (par exemple en matière plastique siliconée) perforable par l'aiguille 21 de tout système approprié d'injection, telle qu'une seringue, ceci à travers la peau du patient schématisée en 23.

Le fond 25 de la capsule pourra notamment être métallique, le reste, et notamment sa paroi latérale 27, pouvant être en matériau isolant électrique.

Pour assurer la distribution du produit reçu de la seringue, le volume 17 communique ici avec le cathéter 3, via un raccord conventionnel 29.

Si, ainsi constituée, l'unité 1 est implantée sans que l'on ait pris de précautions particulières quant à la rigidité du cathéter 3, celui-ci risque de se lover en boucle(s) à l'endroit du coeur, ou, s'il est trop rigide, d'exercer un effet de poussée sur la chambre, risquant de transformer celle-ci en "tunneliseur" sous-cutané.

Pour éviter cela, la paroi de ce dernier est rigidifiée de manière à être plus raide du côté de l'extrémité distale 3b que du côté de son extrémité proximale 3a (où le cathéter est donc plus souple), pour amortir l'effet de poussée sur la chambre 5. (Bien entendu, une autre solution pourrait consister à rigidifier le cathéter en introduisant dans son conduit 9 par exemple un câble métallique de raideur variable, pouvant par exemple présenter un diamètre plus important à son extrémité distale qu'à son extrémité proximale).

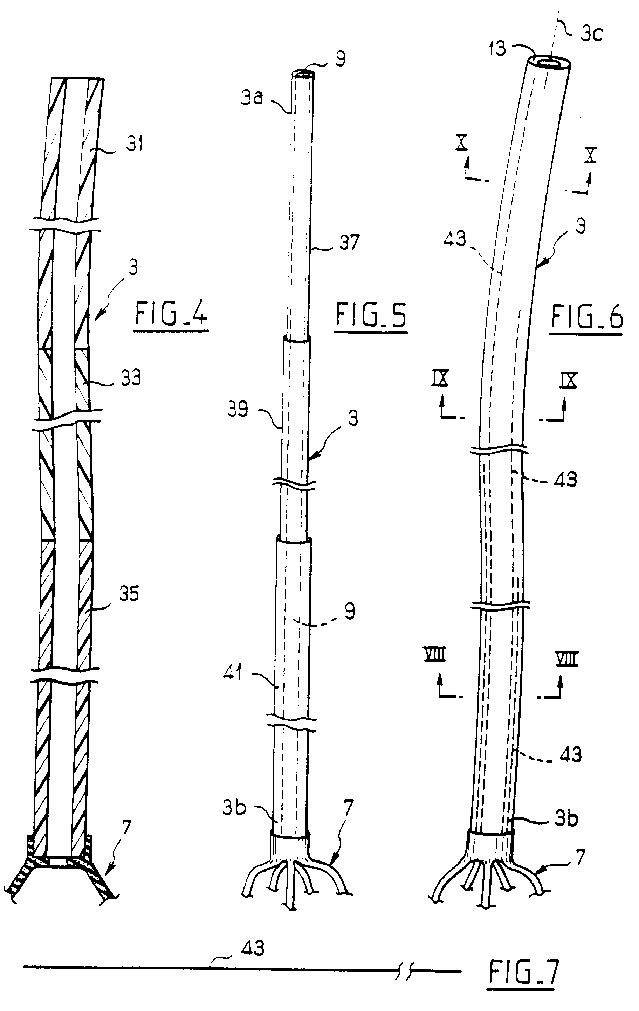

Les figures 4 à 13 montrent (dans le cadre d'une rigidification de paroi, hors conduit interne) plusieurs manières de rigidifier le cathéter, à savoir en l'espèce soit par rigidification structurelle de la paroi elle-même, sans moyens annexes rapportés (figures 4 et 5), soit en utilisant des moyens de rigidification rapportés liés à cette paroi, pouvant notamment y être noyés (solutions des figures 6 à 14).

Sur la figure 4 tout d'abord, la solution retenue consiste à réaliser le cathéter 3 en plusieurs tronçons, en l'espèce au nombre de trois, 31, 33, 35 réunis bout à bout coaxialement, par exemple par collage ou soudage, chaque tronçon de tube étant réalisé en une matière différente ayant sa propre rigidité.

La différence de rigidité entre les tronçons 31, 33, 35 pourrait être obtenue en les réalisant à partir de dosage ou coupage à des taux variables de polyoléfines (tels que polyéthylène) de haute, moyenne et/ ou basse densité, ou encore à partir de polymères séquencés (tels que A-B) dont une séquence serait plus rigide que l'autre et varierait (par exemple poly éther bloc amide : P.E.B.A.).

Une autre solution, illustrée figure 5, consiste à prévoir un cathéter dont l'épaisseur de la paroi va décroissante depuis l'extrémité distale 3b vers l'extrémité proximale 3a.

Dans l'exemple de réalisation représenté, cette variation d'épaisseur a été réalisée en utilisant trois tubes 37, 39, 41 de différentes longueurs et de différentes sections (tant intérieure qu'extérieure), de manière à obtenir un ensemble télescopique, le tube de plus forte section et de plus petite longueur 41 étant fixé au filtre 7 et recevant étroitement le second tube de plus petite section et de plus grande longueur 39 à l'intérieur duquel est emmanché le troisième tube 37 de section encore inférieure et de longueur par contre supérieure.

Si nécessaire, le dernier tube 37 pourra s'étendre jusqu'à l'extrémité distale du tronçon le plus court 41, de telle manière que la section du conduit interne 9 du cathéter soit constante sur toute sa longueur, l'épaisseur de chacun des tubes 37, 39, 41 pouvant être identique.

Sur la figure 6, la rigidification variable du cathéter 3 est obtenue par l'intermédiaire de moyens annexes rapportés, le cathéter étant lui-même constitué d'une seule pièce en matière plastique bio-compatible souple avec une épaisseur de paroi constante d'une extrémité à l'autre.

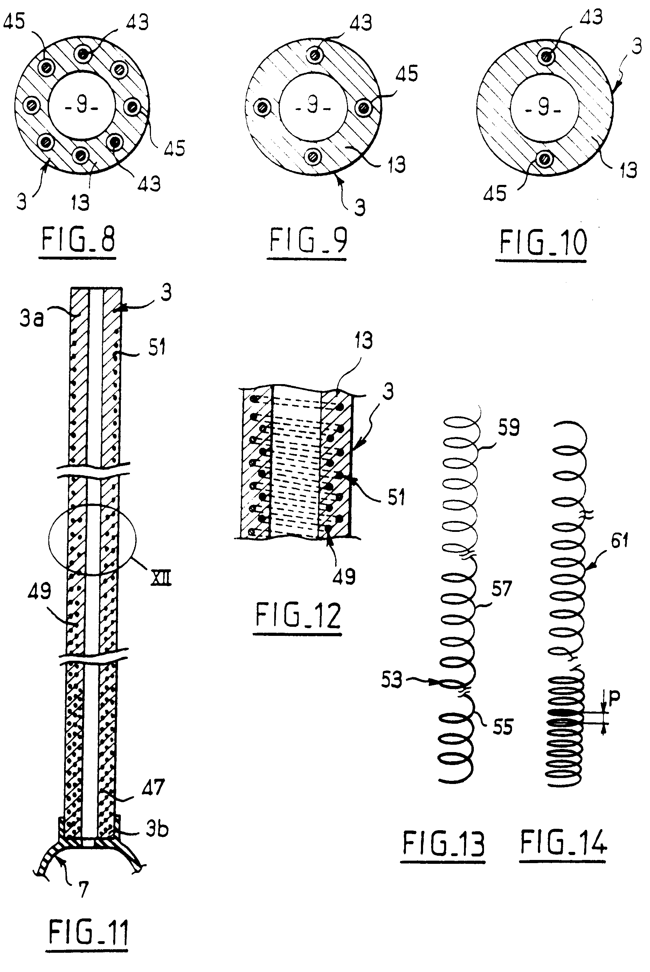

La rigidification est obtenue par l'emploi de fins filaments ou tiges métalliques figurés en 43 sur la figure 7, ces filaments devant être mis en place dans des passages 45 ménagés dans la paroi 13 du cathéter, ces passages, ainsi donc que les tiges, s'étendant sensiblement à partir de l'extrémité distale 3b en direction de l'extrémité proximale 3a.

Pour faire varier cette rigidité, on pourra prévoir d'utiliser des tiges 43 de différentes longueurs.

Dans la version représentée, on a ainsi prévu du côté de l'extrémité distale 3b huit canaux 45 renfermant chacun étroitement une tige 43, ceci sur environ le tiers de la longueur du cathéter (figure 8), le second tiers intermédiaire ne comprenant plus que quatre canaux et quatre tiges (figure 9), le dernier tiers s'étendant jusqu'à l'extrémité proximale 3a, ne renfermant quant à lui que deux canaux 45 avec chacun une tige 43 (figure 10).

Dans la version illustrée figure 11, on utilise non plus des tiges métalliques mais plusieurs ressorts hélicoïdaux noyés à l'intérieur de la paroi du cathéter ici constitué en une seule pièce en matière plastique biocompatible habituel et avec une épaisseur constante.

Dans la version représentée, on a prévu trois tronçons de ressorts hélicoïdaux 47, 49, 51 de différentes longueurs disposés coaxialement les uns autour des autres et donc noyés dans la paroi 13.

Comme l'illustrent respectivement les figures 13 et 14, on pourraît également prévoir de n'employer qu'un seul et unique ressort hélicoïdal, toujours noyé dans l'épaisseur de la paroi du cathéter mais pouvant présenter un pas entre spires et/ ou une épaisseur variable.

Sur la figure 14, le ressort hélicoïdal 61 qui y est représenté présente un pas "p" entre spires qui va croissant dans un sens.

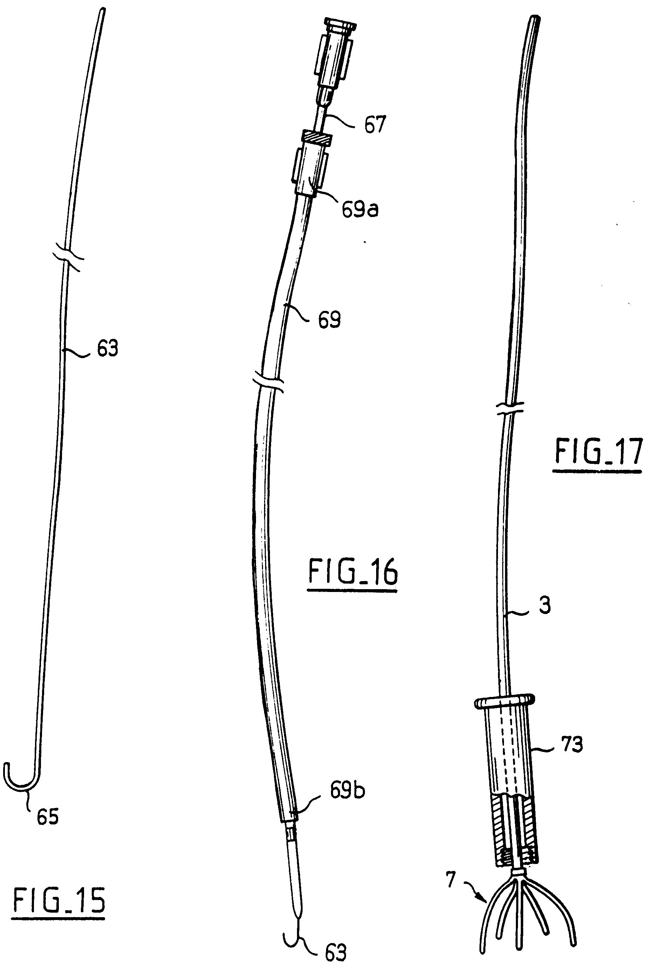

En relation avec les figures 15, 16 et 17, on va maintenant décrire une possible méthode d'implantation de l'unité de filtration 1.

Tout d'abord, on ménage dans le cou une voie d'accès percutanée de la veine jugulaire, l'implantation du filtre étant ici prévue dans la veine cave inférieure.

On introduit ensuite, à travers la voie d'accès, un fil métallique 63 à extrémité recourbée 65.

L'opérateur emmanche alors sur l'extrémité proximale du fil 63 un un mandrin rigide 67 et une gaine 69.

L'opérateur fait ensuite descendre cet ensemble le long du câble 63.

L'opérateur peut alors retirer de la gaine 69 le fil souple 63, ainsi que le mandrin 67 qui l'entoure.

Pré-conditionné, le corps de seringue 73 est ensuite vissé sur l'embout proximal 69a de la gaine 69.

On descend alors dans la gaine le filtre suivi de son cathéter, jusqu'à ce que ledit filtre, parvenu à l'extrémité distale 69b de la gaine, s'expanse.

L'opérateur peut alors retirer la gaine 69 de la veine.

Au bout de l'extrémité libre du cathéter, l'opérateur emmanche ensuite la capsule 5, via le raccord 29, puis enfouit le tout dans le logement et referme la voie d'accès.

A titre indicatif, on notera que le mandrin 67 et la gaine 69 pourront avoir une longueur de l'ordre de 50 à 60 cm, la longueur du cathéter pouvant atteindre 60 à 80 cm, le câble 63 étant de préférence légèrement plus long avec un diamètre pouvant être de l'ordre de 0,5 mm, les diamètres du mandrin 67 et de la gaine pouvant quant à eux être de l'ordre respectivement de 3 et 4 millimètres, celui du cathéter étant lègèrement inférieur, par exemple de l'ordre de 2 mm, sa longueur après sectionnement pouvant atteindre 40 à 50 cm. En particulier, si le cathéter n'est pas utilisé comme moyen de circulation d'un produit, tout moyen équivalent pourra bien entendu être employé, tel qu'un tube flexible en silicone.

## Revendications

1. Dispositif implantable dans le corps d'un patient jusqu'à un vaisseau pour y retenir d'éventuels caillots sanguins comprenant :
- un cathéter (3) présentant un axe (3c) et une longueur dans la direction de cet axe, avec une extrémité proximale (3a) et une extrémité distale opposée (3b), et
- un filtre sanguin (7) fixé à l'extrémité distale du cathéter (3) en maintenant ouverte cette extrémité,
caractérisé en ce que ledit cathéter (3) est raidi de manière variable sur sa longueur, pour être plus raide vers son extrémité distale que vers son extrémité proximale (3a).

2. Dispositif selon la revendication 1, caractérisé en ce que le cathéter, qui présente une paroi entourant un conduit intérieur axial (9), est raidi de manière longitudinalement variable par une rigidification, différente suivant les endroits, de sa dite paroi, à l'extérieur de son dit conduit interne (9) que l'on maintient ainsi libre.

3. Dispositif selon la revendication 1 ou la revendication 2, caractérisé en ce que la rigidité obtenue du cathéter (3) varie longitudinalement par tronçons, en plusieurs endroits, entre son extrémité proximale (3a) et son extrémité distale.

4. Dispositif selon l'une des revendications 2 ou 3, caractérisé en ce qu'il comprend en outre des moyens (5) de distribution d'un produit traitant vers ledit vaisseau, à travers ledit cathéter, et un passage (15) ménagé dans le filtre (7), ces moyens qui comprennent une capsule implantable dans le corps du patient étant accessibles au produit depuis l'extérieur de ce corps.

5. Dispositif selon l'une quelconque des revendications précédentes, caractérisé en ce que la rigidification variable du cathéter (3) est obtenue en faisant varier l'épaisseur de sa paroi.

6. Dispositif selon l'une quelconque des revendications précédentes, caractérisé en ce que le cathéter est constitué de plusieurs tronçons télescopiques (37, 39, 41).

7. Dispositif selon l'une quelconque des revendications précédentes, caractérisé en ce que la rigidification variable est obtenue en utilisant des tronçons de tube (31, 33, 35) réalisés chacun en une matière différente ayant sa propre rigidité, lesdits tronçons étant réunis sensiblement coaxialement, bout à bout, pour constituer le cathéter (3).

8. Dispositif selon l'une des revendications 1 à 4, caractérisé en ce que la rigidification du cathéter est obtenue en utilisant des moyens de rigidification rapportés (43, 47, 49, 51, 53, 61).

9. Dispositif selon la revendication 8, caractérisé en ce que lesdits moyens annexes de rigidification comprennent des moyens à ressorts (47, 49, 51, 53, 61) noyés dans l'épaisseur de la paroi du cathéter.

10. Dispositif selon la revendication 8, caractérisé en ce que lesdits moyens rapportés de rigidification comprennent des tiges (43) de différentes rigidités pouvant être de différentes longueurs et/ ou de différentes sections, ces tiges étant logées dans des passages (45) ménagés à l'intérieur de ladite paroi du cathéter.

## Claims

1. Device implantable in the body of a patient, as far as a vessel, to retain there any blood clots, comprising:
- a catheter (3) having an axis (3c) and a length in the direction of that axis, with a proximal end (3a) and an opposite distal end (3b), and
- a blood filter (7) secured at the distal end of the catheter (3),
characterised in that the catheter (3) is stiffened in a variable manner along its length in order to be stiffer towards its distal end than towards its proximal end (3a).

2. Device according to claim 1, characterised in that the catheter, which has a wall surrounding an axial inner duct (9), is stiffened in a longitudinally variable manner by a rigidification, which varies from place to place, of its wall, outside its inner duct (9) which is thus kept free.

3. Device according to claim 1 or claim 2, characterised in that the rigidity obtained for the catheter (3) varies longitudinally in portions, in several places, between its proximal end (3a) and its distal end.

4. Device according to any one of claims 2 or 3, characterised in that it also comprises means (5) for distributing a treating product towards the vessel, through the catheter, and a passage (15) formed in the filter (7), those means, which comprise a capsule adapted to be implanted in the patient's body, being accessible to the product from outside the body.

5. Device according to any one of the preceding claims, characterised in that the variable rigidification of the catheter (3) is obtained by varying the thickness of its wall.

6. Device according to any one of the preceding claims, characterised in that the catheter is constituted by several telescopic portions (37, 39, 41).

7. Device according to any one of the preceding claims, characterised in that the variable rigidification is obtained by using tube portions (31, 33, 35) each made of a different material having its own rigidity, the portions being joined substantially coaxially, end-to-end, to form the catheter (3).

8. Device according to any one of claims 1 to 4, characterised in that the rigidification of the catheter is obtained by using separate rigidification means (43, 47, 49, 51, 53, 61).

9. Device according to claim 8, characterised in that the additional rigidification means comprise spring means (47, 49, 51, 53, 61) embedded in the thickness of the wall of the catheter.

10. Device according to claim 8, characterised in that the separate rigidification means comprise rods (43) of different rigidities which can have different lengths and/ or different cross-sections, the rods being accommodated in passages (45) formed inside the wall of the catheter.

## Patentansprüche

1. In den Körper eines Patienten bis zu einem Gefäß implantierbare Vorrichtung, um dort mögliche Koagulate zurückzuhalten mit:
- einem Katheter (3), der eine Achse (3c) und eine Länge in der Richtung dieser Achse mit einem proximalen Ende (3a) und einem entgegengesetzten distalen Ende (3b) aufweist, und
- einem Blutfilter (7), der an dem distalen Ende des Katheters (3) befestigt ist,
dadurch gekennzeichnet, daß der Katheter (3) in veränderlicher Weise auf seiner Länge versteift ist, um zu seinem distalen Ende hin steifer zu sein als zu seinem proximalen Ende (3a) hin.

2. Vorrichtung nach Anspruch 1, dadurch gekennzeichnet, daß der Katheter, welcher eine Wand aufweist, die eine innere axiale Leitung (9) umgibt, längs auf veränderliche Weise durch eine Versteifung versteift ist, welche entlang den Stellen seiner Wand außerhalb seiner inneren Leitung (9) unterschiedlich ist, die man so freihält.

3. Vorrichtung nach Anspruch 1 oder Anspruch 2, dadurch gekennzeichnet, daß die erhaltene Steifigkeit des Katheters (3) längs über Abschnitte an mehreren Stellen zwischen seinem proximalen Ende (3a) und seinem distalen Ende variiert.

4. Vorrichtung nach einem der Ansprüche 2 oder 3, dadurch gekennzeichnet, daß sie ferner Mittel (5) für die Verteilung eines Behandlungsproduktes zu dem Gefäß hin quer durch den Katheter, und einen in dem Filter (7) ausgesparten Durchgang (15) aufweist, wobei diese Mittel, welche eine in den Körper des Patienten implantierbare Kapsel aufweisen, für das Produkt von außerhalb dieses Körpers zugänglich sind.

5. Vorrichtung nach einem der vorhergehenden Ansprüche, dadurch gekennzeichnet, daß die variable Versteifung des Katheters (3) dadurch erhalten wird, daß man die Dicke seiner Wand variiert.

6. Vorrichtung nach einem der vorhergehenden Ansprüche, dadurch gekennzeichnet, daß der Katheter aus mehreren zusammenschiebbaren Abschnitten (37, 39, 41) aufgebaut ist.

7. Vorrichtung nach einem der vorhergehenden Ansprüche, dadurch gekennzeichnet, daß die variable Versteifung durch die Verwendung von Rohrabschnitten (31, 33, 35) erhalten wird, von denen jeder aus einem unterschiedlichen Material ausgeführt ist, welches seine eigene Steifigkeit hat, wobei die Abschnitte im wesentlichen koaxial Ende an Ende verbunden sind, um den Katheter (3) zu bilden.

8. Vorrichtung nach einem der Ansprüche 1 bis 4, dadurch gekennzeichnet, daß die Steifigkeit des Katheters durch Verwendung von angestückten Versteifungsmitteln (43, 47, 49, 51, 53, 61) erhalten wird.

9. Vorrichtung nach Anspruch 8, dadurch gekennzeichnet, daß die angefügten Versteifungsmittel Federmittel (47, 49, 51, 53, 61) aufweisen, die in die Dicke der Wand des Katheters völlig eingebettet sind.

10. Vorrichtung nach Anspruch 8, dadurch gekennzeichnet, daß die angestückten Versteifungsmittel Stangen (43) unterschiedlicher Steifigkeiten aufweisen, welche unterschiedliche Längen und/oder unterschiedliche Querschnitte haben können, wobei diese Stangen in den Durchgängen (45) angeordnet sind, die im Inneren der Wand des Katheters ausgespart sind.
